# EUROPEAN PATENT APPLICATION

(11) **EP 2 868 319 A1**
(43) Date of publication of application: **06.05.2015**
(21) Application number: 13810299.1
(22) Date of filing: 25.03.2013
(51) Int. Cl.: A61K 31/497, A61K 9/06, A61K 47/02, A61K 47/12, A61K 47/18, A61K 47/26, A61K 47/30, A61K 47/36, A61K 47/38, A61K 47/42, A61P 25/18

(54) **ARIPIPRAZOLE ORAL PHARMACEUTICAL PREPARATION**

(30) Priority: 29.06.2012 JP 2012147620
(71) Applicant: Maruishi Pharmaceutical Co., Ltd., Osaka 538-0042 (JP)
(72) Inventor: DAIRAKU, Masatake, Osaka-shi Osaka 538-0042 (JP); NAGAHARA, Hironori, Osaka-shi Osaka 538-0042 (JP); MIKI, Atsushi, Osaka-shi Osaka 538-0042 (JP)
(74) Representative: Witte, Weller & Partner Patentanwälte mbB
(86) International application number: PCT/JP2013/058579
(87) International publication number: WO 2014/002553

(57) **Abstract**

An object of the present invention is to provide an oral pharmaceutical preparation of aripiprazole that has suppressed sourness and bitterness, imposes less medication burden on patients, has a higher dissolution rate, and enables patients to easily confirm that they have successfully swallowed the preparation. The present invention relates to an oral pharmaceutical preparation comprising aripiprazole and a gelling agent and having a pH of 4.6 or more.

## Description

### TECHNICAL FIELD

The present invention relates to an oral pharmaceutical preparation of aripiprazole which is suitable for oral administration.

### BACKGROUND ART

In recent years, the number of patients with mental disorders (e.g. schizophrenia and bipolar disorder) is yearly increasing and about 1% of the Japanese are said to be affected by the disorders today. The environmental background factors surrounding the patients include diversifying mental stress coupled with the development of information society. In such social environment, there are various theories as to the causes of the onset of schizophrenia, for example, the dopamine hypothesis, the adrenochrome hypothesis, the glutamate hypothesis, abnormalities in calcineurin genes, genetic defects, the carbonyl stress theory, the developmental disorder hypothesis, the diathesis-stress model, the two-hit theory, the psychogenic theory, the illicit drug theory and the nutritional hypothesis, and the pathogenesis of schizophrenia has been studied from various aspects.

Bipolar disorder (manic-depressive illness) is classified into bipolar I disorder, which is accompanied by a manic state, and bipolar II disorder, which is accompanied by a hypomanic state. Each state of bipolar disorder has some differences from depressive condition (major depressive episode), mixed condition (mixed affective episode), hypomanic condition (hypomanic episode) and major depressive disorder (depression), and has some relevance to other disorders. Thus, the diagnosis and treatment of bipolar disorder are so difficult that even the specialists need to be very careful in diagnosis.

Aripiprazole is 7-{4-[4-(2,3-dichlorophenyl)-1-piperazinyl]-butoxy}-3,4-dih ydro-2(1H)-quinolinone, which is represented by the following formula: 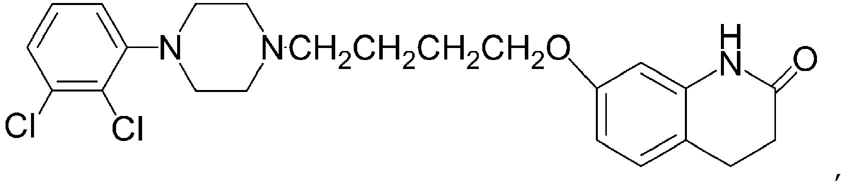 and an antipsychotic drug effective for the treatment of schizophrenia and bipolar disorder. Patients with schizophrenia or bipolar disorder span a wide range of ages from minors to the working generation and also to elderly people. For the treatment of schizophrenia, atypical antipsychotics, which are newly developed drugs having distinctive features and include risperidone, perospirone, olanzapine and quetiapine, are mainly used. In Japan, in addition to these four drugs, aripiprazole, blonanserin, clozapine and paliperidone have also become available, and these eight kinds of atypical antipsychotics are currently used for the treatment.

Among the atypical antipsychotics, olanzapine, quetiapine and aripiprazole reportedly have recurrence preventing effect in addition to antimanic effect. The basics of the medication treatment for bipolar disorder are recurrence prevention by mood stabilizers. The mood stabilizers include lithium carbonate, valproic acid, carbamazepine and lamotrigine, and aripiprazole was also approved as such in 2012. Aripiprazole is sold in various dosage forms to meet consumers' needs, including a tablet, an OD tablet, a powder and an oral solution (for example, Patent Literature 1).

These days, pharmaceutical dosage forms have been diversified, and patients who are aware of a decline or change in their physical functions have come to select a drug that is easier, even if only a little bit, to ingest. Accordingly, many types of easy-to-ingest tablets such as an OD tablet have also been put on the market in Japan.

However, OD tablets reportedly have some disadvantages, such as "OD tablets disintegrate and disperse in the mouth and thus are hard to swallow" and "OD tablets, although believed to have been swallowed, still remain in the mouth," and there is room for improvement.

In the production of liquid preparations of aripiprazole, considering that aripiprazole is highly soluble in an acidic range (pH 4.5 or less), but in a neutral (pH 5) or higher pH range, less soluble and present in a crystalline form, it is difficult to formulate aripiprazole into a liquid preparation in the neutral or higher pH range. Further, in order to ensure the dissolution of aripiprazole from its pharmaceutical preparations, the preparations conventionally need to be produced at an acidic pH (pH 2.5 to 4.5) (See Patent Literature 1).

Oral preparations of aripiprazole produced in the acidic pH range have sourness resulting from the acidic pH and the characteristic bitterness of aripiprazole, and thus need to contain a masking agent such as sweeteners, resulting in an increased volume of the preparation as a whole. For example, a liquid preparation of aripiprazole at a concentration of 12 mg/12 mL is sold (Non Patent Literature 1).

However, this volume is so large as to impose a burden on patients who ingest such a liquid preparation. In addition, this preparation has a pH of about 3 and strong sourness and sweetness, and thus is not preferred by patients who dislike sourness or sweetness.

Therefore, further improvement of aripiprazole preparations has been required.

### CITATION LIST

### Patent Literature

Patent Literature 1: Japanese Patent No. 4401077 Non Patent Literature

Non Patent Literature 1: Interview form of Abilify (registered trademark) oral solution 0.1%, 14th edition (revised in June 2012)

### SUMMARY OF INVENTION

### TECHNICAL PROBLEM

An object of the present invention is to provide an oral pharmaceutical preparation of aripiprazole that has suppressed sourness and bitterness, imposes less medication burden on patients, has a higher dissolution rate, and enables patients to easily confirm that they have successfully swallowed the preparation.

### SOLUTION TO PROBLEM

The present inventors conducted extensive research to solve the above problems, and as a result, found that all the problems can be solved by an oral pharmaceutical preparation of aripiprazole which comprises a gelling agent and has a pH of 4.6 or more. Based on this finding, the present inventors conducted further research and completed the present invention.

That is, the present invention relates to the following.
[1] An oral pharmaceutical preparation comprising aripiprazole and a gelling agent and having a pH of 4.6 or more.
[2] The oral pharmaceutical preparation according to the above [1], wherein the pH of the preparation is adjusted by use of a pharmaceutically usable basic buffering agent at a molar concentration of 0.03 M or less.
[3] The oral pharmaceutical preparation according to the above [2], wherein the basic buffering agent is one or more kinds selected from the group consisting of citrates or their hydrates, malates or their hydrates, phosphates or their hydrates and edetates or their hydrates.
[4] The oral pharmaceutical preparation according to the above [2], wherein the basic buffering agent is one or more kinds selected from the group consisting of sodium citrate, disodium citrate, trisodium citrate, sodium DL-malate, trisodium phosphate, disodium hydrogen phosphate, dipotassium hydrogen phosphate and tetrasodium edetate.
[5] The oral pharmaceutical preparation according to any one of the above [1] to [4], wherein the pH of the preparation is adjusted by use of a pharmaceutically usable basic buffering agent at a molar concentration of 0.03 M or less and a pharmaceutically usable acid.
[6] The oral pharmaceutical preparation according to any one of the above [1] to [5], further comprising a sugar at a concentration of 50 W/W% or less.
[7] The oral pharmaceutical preparation according to any one of the above [1] to [6], wherein the preparation is a semisolid preparation.
[8] The oral pharmaceutical preparation according to any one of the above [1] to [6], wherein the preparation is a liquid preparation.
[9] The oral pharmaceutical preparation according to the above [8], wherein the liquid preparation is a thick liquid preparation.
[10] The oral pharmaceutical preparation according to any one of the above [1] to [9], wherein the gelling agent is one or more kinds selected from the group consisting of gum arabic, gum arabic powder, alginic acid, sodium alginate, propylene glycol alginate, carrageenan, karaya gum powder, carmellose sodium, crystalline cellulose and carmellose sodium, carob bean gum, curdlan, agar, agar powder, xanthan gum, guar gum, psyllium seed gum, gellan gum, purified gelatin, gelatin, tamarind seed gum, tara gum, tragacanth, tragacanth powder, furcellaran, pullulan and pectin.
[11] A method for producing the oral pharmaceutical preparation according to any one of the above [1] to [10], comprising adding a gelling agent to aripiprazole and further adding a pharmaceutically usable basic buffering agent so that the pH of the preparation is adjusted to 4.6 or more.
[12] An oral pharmaceutical composition for use in treatment of mental disorders, the composition comprising aripiprazole and a gelling agent and having a pH of 4.6 or more.
[13] A method for treating mental disorders using an oral pharmaceutical composition comprising aripiprazole and a gelling agent and having a pH of 4.6 or more.

### ADVANTAGEOUS EFFECTS OF INVENTION

The oral pharmaceutical preparation of the present invention has several advantages. For example, unlike OD tablets, the oral pharmaceutical preparation can be sensed as being present in the mouth and also in the throat when being swallowed, thus enabling patients to confirm that they have successfully swallowed the preparation. In addition, the oral pharmaceutical preparation of the present invention does not taste bitter and thus need not contain any masking agent, can reduce the total volume as compared with liquid preparations, and can reduce medication burden on patients. Furthermore, the oral pharmaceutical preparation of the present invention shows the improvement in the delay of aripiprazole dissolution and a favorable dissolution rate in the Japanese Pharmacopoeia (JP) dissolution test using a 1st fluid (a fluid of pH 1.2), and in addition, allows the antipsychotic aripiprazole to be suspended and uniformly dispersed in the fluid.

### BRIEF DESCRIPTION OF DRAWINGS

Fig. 1 shows the results of the dissolution test (in a dissolution medium of pH 1.2) of Example 1 and Comparative Example 1.
Fig. 2 shows the results of the dissolution test (in a dissolution medium of pH 1.2) of Examples 2 and 3 and Comparative Example 2.
Fig. 3 shows the results of the appearance observation of Example 1 after the end of the dissolution test.
Fig. 4 shows the results of the dissolution test (in a dissolution medium of pH 1.2) of Example 4 and Comparative Example 3.
Fig. 5 shows the results of the dissolution test (in a dissolution medium of pH 1.2) of Examples 5 and 6.
Fig. 6 shows the results of the dissolution test (in a dissolution medium of pH 1.2) of Examples 7 and 8.
Fig. 7 shows the results of the dissolution test (in a dissolution medium of pH 1.2) of Examples 9 and 10.
Fig. 8 shows a photograph of the appearances of the jelly-like preparations of Examples 12 to 15. These are jelly-like preparation prototypes which had the active ingredient uniformly dispersed with a turbid appearance and had a favorable dissolution rate in a dissolution medium (test fluid of pH 1.2).
Fig. 9 shows the results of the dissolution test (in a dissolution medium of pH 1.2) of Examples 11 to 15.
Fig. 10 shows the results of the dissolution test (in a dissolution medium of pH 4.0) of Examples 12 to 15.
Fig. 11 shows the results of the dissolution test (in a dissolution medium of pH 1.2) of Examples 16 to 19.
Fig. 12 shows the results of the dissolution test (in a dissolution medium of pH 1.2) of Examples 20 and 21.
Fig. 13 shows the results of the dissolution test (in a dissolution medium of pH 1.2) of Example 22.
Fig. 14 shows the results of the dissolution test (in a dissolution medium of pH 1.2) of Examples 23 and 24.
Fig. 15 shows the results of the dissolution test (in a dissolution medium of pH 1.2) of Examples 25 to 27.
Fig. 16 shows the results of the dissolution test (in a dissolution medium of pH 4.0) of Examples 28 to 32.

### DESCRIPTION OF EMBODIMENTS

The oral pharmaceutical preparation of the present invention comprises aripiprazole and a gelling agent and has a pH of 4.6 or more. In the course of the development of the oral pharmaceutical preparation of the present invention, jelly-like preparation prototypes having the drug in a dissolved state (in an acidic medium) were also produced and examined, but were not fully satisfactory in terms of taste or drug dissolution. The oral pharmaceutical preparation of the present invention is preferably a semisolid or liquid preparation. The semisolid preparation is, although not particularly limited, for example a gelled preparation, a jelly-like preparation, or the like. The gelled preparation means a preparation which contains a water soluble polymer as a gelling agent and is flowable, and the jelly-like preparation means a preparation which contains a water soluble polymer as a gelling agent and is not flowable. The term "gelled" encompasses the form of, for example, thickened liquid, gel, gelee, paste or jam. The liquid preparation is, although not particularly limited, for example a suspension preparation, a thick liquid preparation, a mixed preparation of these, or the like. The thick liquid preparation means a preparation which contains a water soluble polymer etc. as a gelling agent and has a viscosity of 10 to 400 mPa·s, preferably 50 to 400 mPa·s. The viscosity is the value measured by a cone-plate rotational viscometer at a temperature of 20°C and at a shear rate of 50 sec⁻¹ (citation from "Three Levels of Liquid Thickness in 2012 Proposal for the Standardization of Modified Diet for Dysphagic Persons" (the Japanese Society of Dysphagia Rehabilitation)).

The amount (concentration) of aripiprazole in the oral pharmaceutical preparation of the present invention is, although not particularly limited, usually 0.01 to 5.0 W/W%, preferably 0.1 to 3.0 W/W%, and more preferably 0.3 to 1.5 W/W% as a weight ratio in the preparation. The aripiprazole used for the present invention may be in the form of, for example, a free base, a crystalline polymorph of any type (for example, an anhydrous crystal, an anhydrous type B crystal, etc.), a hydrate or a pharmacologically acceptable salt (for example, an acid addition salt etc.). The aripiprazole used for the present invention can be produced by a known method, for example, the method described in JP-A 2-191256, and the methods described in Japanese patent No. 4284524, Japanese patent No. 4614870, etc.

The weight of the preparation of the present invention varies depending on the concentration of the drug, but can be optimally adjusted as needed. The amount of the preparation per dose is not particularly limited, but in terms of ease of ingestion for patients, a lower amount is preferable. For example, 20 g or less is preferable, 10 g or less is more preferable, and 5 g or less is still more preferable because this amount is allowed to be put into the mouth at once and easy to ingest without choking or spilling.

In one embodiment of the present invention, the aripiprazole-containing oral pharmaceutical preparation of the present invention can be provided as a gelled preparation or a jelly-like preparation each comprising a gelling agent. In another embodiment of the present invention, the aripiprazole-containing oral pharmaceutical preparation of the present invention can be provided as a suspension preparation or a thick liquid preparation each comprising a gelling agent. Examples of the gelling agent include gum arabic, gum arabic powder, alginic acid, sodium alginate, propylene glycol alginate, carrageenan ( 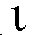 carrageenan, κ carrageenan, etc.), karaya gum powder, carmellose sodium, crystalline cellulose and carmellose sodium, carob bean gum, curdlan, agar, agar powder, xanthan gum, guar gum, psyllium seed gum, gellan gum, purified gelatin, gelatin, tamarind seed gum, tara gum, tragacanth, tragacanth powder, furcellaran, pullulan and pectin. Depending on the dosage form of the present invention, these gelling agents may be used alone or as a mixture of two or more kinds. In the case of a suspension preparation, alginic acid, sodium alginate, propylene glycol alginate, crystalline cellulose and carmellose sodium, etc. can be preferably used as a gelling agent. Among the above-listed gelling agents, sodium alginate, carrageenan, carob bean gum, agar powder, xanthan gum and pectin are preferable, and carrageenan and carob bean gum are particularly preferable. In the mechanism of gelation by gelling agents, their water soluble polymer molecules are hydrated, thereby extended and randomly entangled with each other, resulting in forming a gel. The amount of the gelling agent in the oral pharmaceutical preparation of the present invention is not particularly limited unless the effects of the present invention are hindered.

The pH of the oral pharmaceutical preparation of the present invention is preferably 4.6 or more, more preferably 5.0 or more, and still more preferably 6 or more. The upper limit of the pH is not particularly limited, but considering that strong alkaline preparations potentially cause mouth and esophagus irritation and taste unpleasant due to its harsh flavor, the pH is preferably 9 or less, more preferably 8 or less, and still more preferably 7 or less. In view of the characteristics and producibility of the preparation, an appropriate pH can be set to the value of 4.6 or more.

One embodiment of the semisolid preparation of the present invention is, for example, a jelly-like preparation. Hereinafter, the jelly-like preparation will be described.

For adjustment of the pH to 4.6 or more in the jelly-like preparation, a pharmaceutically usable basic buffering agent is used. In the present invention, in addition to the basic buffering agent, a pharmaceutically usable acid may also be used for the pH adjustment. A combined use of the basic buffering agent and the acid is preferable because the combination allows high drug dissolution. In the present invention, depending on the form of aripiprazole, the amounts of the basic buffering agent and the acid may be adjusted as long as the pH falls within the range specified in the present invention. For example, in the case where an acid addition salt of aripiprazole is used, the adjustment can be performed by appropriately reducing the amount of the acid.

The basic buffering agent used in the jelly-like preparation is not particularly limited unless the effects of the present invention are hindered, and may be any basic buffering agent that can be pharmaceutically used. Examples of the basic buffering agent include citrates or their hydrates, malates or their hydrates, phosphates or their hydrates, edetates or their hydrates and lactates or their hydrates. The above-listed salts are not particularly limited as long as they are pharmacologically acceptable salts, and the examples include pharmacologically acceptable metal salts, ammonium salts, organic amine addition salts and amino acid addition salts. The pharmacologically acceptable metal salts are not particularly limited and the examples include alkali metal salts such as sodium salts and potassium salts; alkaline earth metal salts such as calcium salts and magnesium salts; aluminum salts; and zinc salts. The pharmacologically acceptable organic amine addition salts are not particularly limited and the examples include morpholine salts and piperidine salts. The pharmacologically acceptable amino acid addition salts are not particularly limited and the examples include lysine salts, glycine salts and phenylalanine salts. These salts may be anhydrides or hydrates.

The citrates or their hydrates are not particularly limited and the examples include sodium citrate and disodium citrate. The malates or their hydrates are not particularly limited and the examples include sodium DL-malate. The phosphates or their hydrates are not particularly limited and the examples include trisodium phosphate, disodium hydrogen phosphate and dipotassium hydrogen phosphate. The edetates or their hydrates are not particularly limited and the examples include tetrasodium edetate. The lactates or their hydrates are not particularly limited and the examples include sodium lactate. Among the above-listed basic buffering agents, sodium citrate, disodium citrate, trisodium citrate, sodium DL-malate, trisodium phosphate, disodium hydrogen phosphate, dipotassium hydrogen phosphate and tetrasodium edetate are preferable, and sodium citrate, disodium citrate, trisodium citrate, disodium hydrogen phosphate, dipotassium hydrogen phosphate and tetrasodium edetate are particularly preferable. These basic buffering agents may be used alone or as a mixture of two or more kinds.

The acid is not particularly limited as long as it can be pharmaceutically used, and the examples include organic acids (for example, acetic acid, trifluoroacetic acid, formic acid, propionic acid, fumaric acid, maleic acid, succinic acid, tartaric acid, D-tartaric acid, citric acid, DL-malic acid, lactic acid, oxalic acid, benzoic acid, besylic acid, ascorbic acid, methanesulfonic acid, ethanesulfonic acid, benzenesulfonic acid, toluenesulfonic acid, edetic acid, etc.); and inorganic acids (for example, hydrochloric acid, phosphoric acid, hydrobromic acid, hydroiodic acid, nitric acid, sulfuric acid, etc.). The acid may be in the form of a pharmacologically acceptable salt as long as it can be used as an acid. The specifics of the salt are as described above for the basic buffering agent, and the examples include edetates or their hydrates (for example, disodium edetate etc.), phosphates or their hydrates (for example, potassium dihydrogen phosphate, sodium dihydrogen phosphate, etc.), tartrates or their hydrates (for example, disodium DL-tartrate, potassium hydrogen tartrate, etc.), and fumarates or their hydrates (for example, monosodium fumarate etc.).

Among the above-listed acids, hydrochloric acid, citric acid, DL-malic acid, phosphoric acid, edetic acid, potassium dihydrogen phosphate, sodium dihydrogen phosphate and disodium edetate are preferable, and hydrochloric acid, citric acid, DL-malic acid, edetic acid, sodium dihydrogen phosphate and disodium edetate are particularly preferable. These acids may be used alone or as a mixture of two or more kinds.

In cases where a combination of the basic buffering agent and the acid is used, it is preferable to combine one or more basic buffering agents selected from the group consisting of sodium citrate, disodium citrate, trisodium citrate, sodium DL-malate, trisodium phosphate, disodium hydrogen phosphate, dipotassium hydrogen phosphate and tetrasodium edetate with one or more acids selected from the group consisting of hydrochloric acid, citric acid, DL-malic acid, phosphoric acid, edetic acid, potassium dihydrogen phosphate, sodium dihydrogen phosphate and disodium edetate; and it is more preferable to combine one or more basic buffering agents selected from the group consisting of sodium citrate, disodium citrate, trisodium citrate, disodium hydrogen phosphate, dipotassium hydrogen phosphate and tetrasodium edetate with one or more acids selected from the group consisting of hydrochloric acid, citric acid, DL-malic acid, edetic acid, sodium dihydrogen phosphate and disodium edetate.

The molar concentration of the basic buffering agent used in the jelly-like preparation is preferably 0.03 M or less for favorable drug dissolution from the jelly-like preparation, more preferably 0.02 M or less, still more preferably 0.01 M or less, and particularly preferably 0.005 M or less. The lower limit of the concentration of the basic buffering agent is not particularly limited as long as the buffering effect can be obtained. For example, the lower limit may be about 0.0001 M, and as long as the buffering effect can be obtained, the lower limit may be less than 0.0001 M. Too high concentration of the salt may cause insufficient dissolution, resulting in a partial amount of aripiprazole remaining undissolved. In the present invention, in the case where a combination of the basic buffering agent and the acid is used, the balance between the amounts of the basic buffering agent and the acid is also important. When the basic buffering agent is 1 M, the molar concentration of the acid is preferably about 0.001 to 0.95 M, and more preferably about 0.01 to 0.85 M.

In the jelly-like preparation of the present invention, a sugar is preferably used in order to control the physical properties such as jelly strength and smoothness, and the gelling and dissolution temperatures as desired.

The sugar used in the jelly-like preparation is not particularly limited and the examples include D-sorbitol, D-sorbitol solution, mannitol, erythritol, xylitol, maltitol, maltitol solution, lactitol, trehalose, reduced maltose syrup, sucrose and purified sucrose. Preferred are D-sorbitol, D-sorbitol solution, xylitol, reduced maltose syrup and purified sucrose. These sugars may be used alone or as a mixture of two or more kinds.

In the present invention, in order that the oral pharmaceutical preparation retains the physical properties of a jelly-like preparation, a sugar is preferably used. Considering that the weight of the sugar in the preparation affects the dissolution behavior in a dissolution medium of pH 1.2 (a 1st fluid for the JP dissolution test), the concentration of the sugar in the sugar-containing jelly-like preparation is preferably 50 W/W% or less, more preferably 45 W/W% or less, and still more preferably 40 W/W% or less. The lower limit is at least 5 W/W% for control of the physical properties and the gelling and dissolution temperatures, preferably 10 W/W%, and more preferably 15 W/W%.

In the present invention, in addition to an appropriate amount of the sugar, a polyhydric alcohol may also be added in producing the jelly-like preparation. Polyhydric alcohols impart moisture and smoothness to the product and thereby the resulting jelly-like preparation can have good texture. The polyhydric alcohol is not particularly limited unless the effects of the present invention are hindered, and the examples include glycerin, ethylene glycol, butylene glycol, propylene glycol, macrogol, polypropylene glycol and polyoxyethylene(160)polyoxypropylene(30)glycol (also called Pluronic F68). These polyhydric alcohols may be used alone or as a mixture of two or more kinds. The amount of the polyhydric alcohol in the oral pharmaceutical preparation of the present invention is not particularly limited unless the effects of the present invention are hindered.

The sugar content (Brix) of the preparation is preferably 50% or less, more preferably 45% or less, and still more preferably 40% or less. When the sugar content is in the above range, a jelly-like preparation having a favorable dissolution rate and a good and smooth texture can be obtained.

The jelly-like preparation of the present invention may contain a sweetener if needed. The sweetener is not particularly limited and the examples include sucralose, aspartame, high-fructose corn syrup, reduced maltose syrup, powdered reduced maltose syrup, saccharin, sodium saccharin, stevia, thaumatin, erythritol, sorbitol, sorbitol solution, lactose, honey, xylitol, glycerin, concentrated glycerin, maltitol and maltitol solution. Preferred is sucralose. These sweeteners may be used alone or as a mixture of two or more kinds. The amount of the sweetener in the oral pharmaceutical preparation of the present invention is not particularly limited unless the effects of the present invention are hindered.

The jelly-like preparation of the present invention does not taste bitter or sour and thus need not contain any flavoring agent, but if needed, may be flavored with, for example, a flavoring agent preferred by children. Examples of such a flavoring agent include an orange flavor, a banana flavor, a strawberry flavor, a vanilla flavor and various flavor essences. These flavoring agents may be used alone or as a mixture of two or more kinds. The amount of the flavoring agent in the oral pharmaceutical preparation of the present invention is not particularly limited unless the effects of the present invention are hindered.

The jelly-like preparation of the present invention may contain a preservative if needed. The preservative is not particularly limited as long as it is for pharmaceutical use, and the examples include methyl p-hydroxybenzoate, ethyl p-hydroxybenzoate, propyl p-hydroxybenzoate, isopropyl p-hydroxybenzoate, butyl p-hydroxybenzoate, isobutyl p-hydroxybenzoate, benzyl p-hydroxybenzoate, sodium benzoate, benzoic acid, benzyl benzoate, benzalkonium chloride, cetylpyridinium chloride, benzethonium chloride, aminoethylsulfonic acid, sorbic acid and sorbates. These preservatives may be used alone or as a mixture of two or more kinds. The addition of such preservatives enhances the storage stability of the product and thus enables long-term prevention of product deterioration (microbial contamination). The amount of the preservative in the oral pharmaceutical preparation of the present invention is not particularly limited unless the effects of the present invention are hindered.

The jelly-like preparation of the present invention can be produced under a manufacturing environment suitable for pharmaceutical production, by use of apparatus in which an appropriate production machine equipped with a stirring device, an appropriate stirring machine equipped with a heating and cooling system, and an appropriate filling and packaging machine are combined at a pharmaceutical level. The production scale can be selected as appropriate in consideration of productivity, efficiency and profitability.

Another embodiment of the semisolid preparation of the present invention is, for example, a gelled preparation. Hereinafter, the gelled preparation will be described.

For adjustment of the pH to 4.6 or more in the gelled preparation, a pharmaceutically usable basic buffering agent is used as is the case with the jelly-like preparation. In the present invention, in addition to the basic buffering agent, a pharmaceutically usable acid may also be used for the pH adjustment. A combined use of the basic buffering agent and the acid is preferable because the combination allows high drug dissolution. As is the case with the jelly-like preparation, in the gelled preparation of the present invention, depending on the form of aripiprazole, the amounts of the basic buffering agent and the acid may be adjusted as long as the pH falls within the range specified in the present invention. The basic buffering agent, the acid and the combination thereof that can be used in the gelled preparation are the same as those exemplified in the jelly-like preparation.

The molar concentration of the basic buffering agent used in the gelled preparation is preferably 0.03 M or less for favorable drug dissolution from the gelled preparation, more preferably 0. 02 M or less, still more preferably 0.01 M or less, and particularly preferably 0.005 M or less. The lower limit of the concentration of the basic buffering agent is not particularly limited as long as the buffering effect can be obtained. For example, the lower limit may be about 0.0001 M, and as long as the buffering effect can be obtained, the lower limit may be less than 0.0001 M. Too high concentration of the salt may cause insufficient dissolution, resulting in a partial amount of aripiprazole remaining undissolved. In the present invention, in the case where a combination of the basic buffering agent and the acid is used, the balance between the amounts of the basic buffering agent and the acid is also important. When the basic buffering agent is 1 M, the molar concentration of the acid is preferably about 0.001 to 0.95 M, and more preferably about 0.01 to 0.85 M.

In the gelled preparation of the present invention, a sugar is preferably used in order to control the physical properties such as smoothness, and the gelling and dissolution temperatures as desired.

The sugar used in the gelled preparation and its concentration are the same as those exemplified in the jelly-like preparation.

In the present invention, in order that the oral pharmaceutical preparation retains the physical properties of a gelled preparation, a sugar is preferably used. Considering that the weight of the sugar in the preparation affects the dissolution behavior in a dissolution medium of pH 1.2 (a 1st fluid for the JP dissolution test), the concentration of the sugar in the sugar-containing gelled preparation is preferably 50 W/W% or less, more preferably 45 W/W% or less, and still more preferably 40 W/W% or less. The lower limit is at least 5 W/W% for control of the physical properties and the gelling and dissolution temperatures, preferably 10 W/W%, and more preferably 15 W/W%.

In the present invention, in addition to an appropriate amount of the sugar, a polyhydric alcohol may also be added in producing the gelled preparation. Polyhydric alcohols impart moisture and smoothness to the product and thereby the resulting gelled preparation can have good texture. The polyhydric alcohol used in the gelled preparation is the same as that exemplified in the jelly-like preparation. The sugar content (Brix) of the preparation is also the same as that of the jelly-like preparation.

The gelled preparation of the present invention may contain a sweetener, a flavoring agent and/or a preservative if needed. The sweetener, the flavoring agent and the preservative used in the gelled preparation is the same as those exemplified in the jelly-like preparation.

The gelled preparation of the present invention can be produced by using an appropriate gelling agent selected by the skilled person from the above-listed gelling agents based on the common technical knowledge. For example, the gelled preparation can be obtained by a combined use of 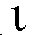 carrageenan and carob bean gum without the use of κ carrageenan. The water soluble gelling polymers as the gelling agent used herein include synthetic polymers, semi-synthetic polymers (polymers obtained through, for example, molecular weight adjustment by acid/base treatment or heat treatment, or solubilization by addition of a metal salt), and naturally-occurring polymers (purified polymers from animals and plants, and microorganisms). These polymers can be divided into the following two types: one type which solidifies to form a jelly when used alone or in a combination of two or more kinds, or together with a metal salt; and the other type which forms a gel as the thickness increases in a concentration depending manner.

These characteristics can be advantageously utilized to produce the desired jelly-like preparation or the thick liquid preparation described below. The water soluble polymers may be used alone or in a combination of two or more kinds as needed. However, some of them become very poor in dispersibility and solubility in water once solidified, and thus, attention must be paid to the use of the water soluble polymers in the pharmaceutical design of the jelly-like preparation and the thick liquid preparation. Therefore, by appropriately adjusting a combination of the water soluble polymers and their amounts, the jelly-like preparation and the thick liquid preparation of aripiprazole can be produced.

One embodiment of the liquid preparation of the present invention is, for example, a thick liquid preparation. Hereinafter, the thick liquid preparation will be described.

For adjustment of the pH to 4.6 or more in the thick liquid preparation, a pharmaceutically usable basic buffering agent is used as is the case with the jelly-like preparation. In the present invention, in addition to the basic buffering agent, a pharmaceutically usable acid may also be used for the pH adjustment. A combined use of the basic buffering agent and the acid is preferable because the combination allows high drug dissolution. As is the case with the jelly-like preparation, in the thick liquid preparation of the present invention, depending on the form of aripiprazole, the amounts of the basic buffering agent and the acid may be adjusted as long as the pH falls within the range specified in the present invention. The basic buffering agent, the acid and the combination thereof that can be used in the thick liquid preparation are the same as those exemplified in the jelly-like preparation.

The molar concentration of the basic buffering agent used in the thick liquid preparation is preferably 0.03 M or less for favorable drug dissolution from the thick liquid preparation, more preferably 0.02 M or less, still more preferably 0.01 M or less, and particularly preferably 0.005 M or less. The lower limit of the concentration of the basic buffering agent is not particularly limited as long as the buffering effect can be obtained. For example, the lower limit may be about 0.0001 M, and as long as the buffering effect can be obtained, the lower limit may be less than 0.0001 M. Too high concentration of the salt may cause insufficient dissolution, resulting in a partial amount of aripiprazole remaining undissolved. In the present invention, in the case where a combination of the basic buffering agent and the acid is used, the balance between the amounts of the basic buffering agent and the acid is also important. When the basic buffering agent is 1 M, the molar concentration of the acid is preferably about 0.001 to 0.95 M, and more preferably about 0.01 to 0.85 M.

In the thick liquid preparation of the present invention, a sugar is preferably used in order to improve the smoothness in combination with the water soluble polymer and to improve the ease of ingestion by imparting sweet taste.

The sugar used in the thick liquid preparation and its concentration are the same as those exemplified in the jelly-like preparation.

In the present invention, in order that the oral pharmaceutical preparation retains the physical properties of a thick liquid preparation, a sugar is preferably used. Considering that the weight of the sugar in the preparation affects the dissolution behavior in a dissolution medium of pH 1.2 (a 1st fluid for the JP dissolution test), the concentration of the sugar in the sugar-containing thick liquid preparation is preferably 50 W/W% or less, more preferably 45 W/W% or less, and still more preferably 40 W/W% or less. The lower limit is at least 5 W/W% for control of the physical properties and the gelling and dissolution temperatures, preferably 10 W/W%, and more preferably 15 W/W%.

In the present invention, in addition to an appropriate amount of the sugar, a polyhydric alcohol may also be added in producing the thick liquid preparation. Polyhydric alcohols impart moisture and smoothness to the product and thereby the resulting thick liquid preparation can have good texture. The polyhydric alcohol used in the thick liquid preparation is the same as that exemplified in the jelly-like preparation. The sugar content (Brix) of the preparation is also the same as that of the jelly-like preparation.

The thick liquid preparation of the present invention may contain a sweetener, a flavoring agent and/or a preservative if needed. The sweetener, the flavoring agent and the preservative used in the thick liquid preparation is the same as those exemplified in the jelly-like preparation.

The thick liquid preparation of the present invention is preferably used for the prevention of pulmonary aspiration in psychotic patients whose swallowing function is further reduced due to antipsychotic medication.

One embodiment of the liquid preparation of the present invention is, for example, a suspension preparation having aripiprazole in an undissolved state. Hereinafter, the suspension preparation will be described.

For adjustment of the pH to 4.6 or more in the suspension preparation, a pharmaceutically usable basic buffering agent is used as is the case with the jelly-like preparation. In the present invention, in addition to the basic buffering agent, a pharmaceutically usable acid may also be used for the pH adjustment. A combined use of the basic buffering agent and the acid is preferable because the combination allows high drug dissolution. As is the case with the jelly-like preparation, in the suspension preparation of the present invention, depending on the form of aripiprazole, the amounts of the basic buffering agent and the acid may be adjusted as long as the pH falls within the range specified in the present invention. The basic buffering agent, the acid and the combination thereof that can be used in the suspension preparation are the same as those exemplified in the jelly-like preparation.

The molar concentration of the basic buffering agent used in the suspension preparation is preferably 0.03 M or less for favorable drug dissolution from the suspension preparation, more preferably 0.02 M or less, still more preferably 0.01 M or less, and particularly preferably 0.005 M or less. The lower limit of the concentration of the basic buffering agent is not particularly limited as long as the buffering effect can be obtained. For example, the lower limit may be about 0.0001 M, and as long as the buffering effect can be obtained, the lower limit may be less than 0.0001 M. Too high concentration of the salt may cause insufficient dissolution, resulting in a partial amount of aripiprazole remaining undissolved. In the present invention, in the case where a combination of the basic buffering agent and the acid is used, the balance between the amounts of the basic buffering agent and the acid is also important. When the basic buffering agent is 1 M, the molar concentration of the acid is preferably about 0.001 to 0.95 M, and more preferably about 0.01 to 0.85 M.

In the suspension preparation of the present invention, a sugar is preferably used in order to improve the smoothness in combination with the water soluble polymer and to improve the ease of ingestion by imparting sweet taste.

The sugar used in the suspension preparation and its concentration are the same as those exemplified in the jelly-like preparation.

In the present invention, in order that the oral pharmaceutical preparation retains the physical properties of a suspension preparation, a sugar is preferably used. Considering that the weight of the sugar in the preparation affects the dissolution behavior in a dissolution medium of pH 1.2 (a 1st fluid for the JP dissolution test), the concentration of the sugar in the sugar-containing suspension preparation is preferably 50 W/W% or less, more preferably 45 W/W% or less, and still more preferably 40 W/W% or less. The lower limit is at least 5 W/W% for control of the physical properties and the gelling and dissolution temperatures, preferably 10 W/W%, and more preferably 15 W/W%.

In the present invention, in addition to an appropriate amount of the sugar, a polyhydric alcohol may also be added in producing the suspension preparation. Polyhydric alcohols impart moisture and smoothness to the product and thereby the resulting suspension preparation can have good texture. The polyhydric alcohol used in the suspension preparation is the same as that exemplified in the jelly-like preparation. The sugar content (Brix) of the preparation is also the same as that of the jelly-like preparation.

The suspension preparation of the present invention may contain a sweetener, a flavoring agent and/or a preservative if needed. The sweetener, the flavoring agent and the preservative used in the suspension preparation is the same as those exemplified in the jelly-like preparation.

The suspension preparation of the present invention is preferably used for the prevention of pulmonary aspiration in psychotic patients whose swallowing function is further reduced due to antipsychotic medication.

The oral pharmaceutical preparation of the present invention obtainable as described above has a dissolution rate (%) of preferably 50% or more at 30 minutes from the start of the dissolution test described below, more preferably 60% or more at 30 minutes from the start of the test, and still more preferably 70% or more at 30 minutes from the start of the test.

The oral pharmaceutical preparation of the present invention can be used for a mental disorder. The mental disorder is not particularly limited and the examples include schizophrenia and bipolar disorder. The bipolar disorder (manic-depressive illness) is not particularly limited and the examples include bipolar I disorder, which is accompanied by a manic state, and bipolar II disorder, which is accompanied by a hypomanic state.

### EXAMPLES

Hereinafter, the present invention will be illustrated in more detail by examples, but is not limited thereto. Many variations can be made by the person having ordinary knowledge in the art within the scope of the technical idea of the present invention. The amounts of the ingredients in Examples and Comparative Examples are expressed in percentage on a mass basis (W/W%).

The molecular weights of the compounds used in the following Examples and Comparative Examples are described below, and all the compounds are products for drug preparation complying with the standards of Japanese pharmacopoeia. The disodium hydrogen phosphate used is in the form of a dodecahydrate (molecular weight: 358.14), the sodium citrate used is in the form of a dihydrate (molecular weight: 294.1), the DL-malic acid used is in the form of an anhydride (molecular weight: 134.09), the citric acid used is in the form of an anhydride (molecular weight: 192.12), and the sodium dihydrogen phosphate used is in the form of a dihydrate (molecular weight: 156.01).

### <Production Apparatus for Prototype Preparations>

The experimental scale production of prototype preparations of the present invention was performed using an apparatus for repetitive small-scale (beaker-scale) experiments, which is capable of heating, stirring and cooling the content and of preventing the evaporation of water by heating, and is devised to allow accurate production of prototypes. Specifically, prototype preparations were produced on a 100-g scale using a production apparatus composed of an airtight heat-resistant glass vessel, a magnetic stirrer (R-1, manufactured by Toyosangyo K.K.) as a stirring device, and a water bath device capable of heating and cooling (B-UP, manufactured by Toyosangyo K.K.). The ingredients were weighed out with electronic balances (PR1203, manufactured by Mettler-Toledo International Inc.; and AW120, manufactured by Shimadzu Corporation).

### <Examples 1 to 3 and Comparative Examples 1 and 2>

The ingredients shown in Table 1 below were precisely weighed out. A powder mixture of 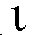 carrageenan, κ carrageenan and carob bean gum was added to a sugar (D-sorbitol). To this, a basic buffering agent (disodium hydrogen phosphate) and an acid (DL-malic acid or citric acid) were added, and a preservative (propyl p-hydroxybenzoate) was further added. Further, purified water and concentrated glycerin were added and the vessel was tightly capped. The mixture was heated with stirring in a hot water bath to 80 to 85°C for complete dissolution. After the temperature reached 80°C, with this temperature maintained for 1 hour, the resulting jelly base was sterilized while stirred so gently as not to form bubbles etc. To the jelly base, aripiprazole weighed in the amount shown in Table 1 below was added and dispersed with mixing, and the mixture was stirred in another hot water bath at 50 to 60°C to give a dispersion. While this temperature (50 to 60°C) was maintained, the dispersion was distributed into previously prepared packaging containers (aluminum stick packs or clear PET containers) so that each container contained a single dosage unit (containing 12 mg of aripiprazole in a 4-g jelly-like preparation). The containers were then sealed with a sealer and cooled for solidification to give packaged products of a jelly-like preparation.

pH test: For pH measurement, the preparation was brought into direct contact with the sensor part of a pH meter (manufactured by HORIBA, Ltd., trade name: Twin pH, model: B-212) and the pH value was read.

Sugar content (Brix) test: For sugar content measurement, the preparation was brought into direct contact with the sensor part of PAL-J manufactured by ATAGO CO., LTD. and the percentage value of sugar content (Brix%) was read.

Appearance observation: The production process of the preparation and the conditions of the packaged product were visually observed, and if needed, photographed with a digital camera (Canon SX210 IS) and saved as an observation record. Similarly in a dissolution test, the conditions such as the degree of dissolution and the degree of undissolution were observed, and if needed, photographed with the same camera as above and saved as an observation record. For comparison of Examples 28 to 32, the respective packaged products were stored at 50°C for 3 days and subsequently at 40°C for 7 days, and their appearances were observed in comparison with the controls in which the packaged products were stored at 25°C for 7 days to examine the difference in the sedimentation of the drug.

Viscosity measurement test: For viscosity measurement, a TOKI SANGYO viscometer (TV-20) equipped with a rotor 3°×R12 was used. The viscosities were measured at rotation speeds of 10 rpm to 100 rpm, the measured viscosities η (mPa·s) were plotted against the shear rates D (⁻S) to produce a viscosity curve (log-log scale), and the viscosity value at a shear rate of 50 sec⁻¹ was calculated by interpolation using an approximate function of the viscosity curve.

Dissolution test: Drug dissolution was analyzed in real time using a dissolution testing system composed of a dissolution tester NTR-6200AC manufactured by Toyama Sangyo Co., Ltd. connected with a looping pump (AUTO-SAMPLER-W manufactured by Toyama Sangyo Co., Ltd.) and with a UV meter UV-1800 manufactured by Shimadzu Corporation.
Dissolution test conditions: Paddle method, 50 rpm
Test temperature: 37 ± 1°C
Volume of dissolution medium: 900 mL
pH of dissolution media: A 1st fluid for the JP dissolution test (pH 1.2), a diluted McIlvaine's buffer solution (pH 4.0), and a 2nd fluid for the JP dissolution test (pH 6.8)

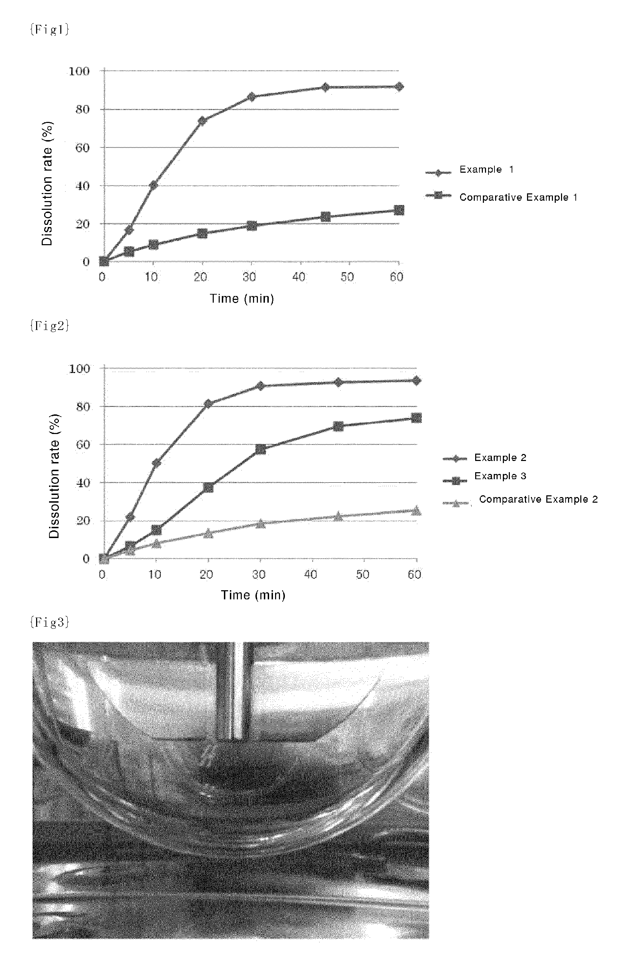

For comparison of the samples of Examples 1 to 3 and Comparative Examples 1 and 2, the results of the pH test and the Brix test (the Brix test was performed on Example 1 only) are shown in Table 1 below, and the results of the dissolution test (in the dissolution medium of pH 1.2) are shown in Figs. 1 and 2. The results of the appearance observation of the sample of Example 1 after the end of the dissolution test are shown in Fig. 3.

**Table 1**

| Ingredients | Example 1 | Comparative Example 1 | Example 2 | Example 3 | Comparative Example 2 |
|---|---|---|---|---|---|
| Aripiprazole | 0.30 | 0.30 | 0.30 | 0.30 | 0.30 |
| 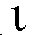 Carrageenan | 0.40 | 0.40 | 0.40 | 0.40 | 0.40 |
| κ Carrageenan | 0.18 | 0.18 | 0.18 | 0.18 | 0.18 |
| Carob bean gum | 0.18 | 0.18 | 0.18 | 0.18 | 0.18 |
| D-sorbitol | 30.0 | 30.0 | 30.0 | 30.0 | 30.0 |
| Glycerin | 10.0 | 10.0 | 10.0 | 10.0 | 10.0 |
| Pluronic F68 | 0.75 | 0.75 | 0.75 | 0.75 | 0.75 |
| DL-malic acid | 0.05 | 0.20 | - | - | - |
| Citric acid | - | - | 0.050 | 0.10 | 0.20 |
| Disodium hydrogen phosphate | 0.36 | 1.44 | 0.36 | 0.72 | 1.44 |
| Propyl p-hydroxybenzoate | 0.015 | 0.015 | 0.015 | 0.015 | 0.015 |
| Purified water | 57.765 | 56.535 | 57.235 | 57.355 | 56.535 |
| Total | 100 | 100 | 100 | 100 | 100 |
| pH | 6.2 | 6.1 | 6.3 | 6.3 | 6.3 |
| Brix% | 39.5 | - | - | - | - |

(In the table, the amounts of the ingredients are expressed in grams.)

DL-malic acid was added to a 0.01 M disodium hydrogen phosphate solution, and the resulting solution was used for the pH adjustment of the preparation to about 6 (Example 1). As shown in Fig. 1, when these two ingredients were used in 4 times (Comparative Example 1) the amounts used in Example 1, the dissolution rate was reduced. In Fig. 2, citric acid was added to a 0.04 M disodium hydrogen phosphate solution, and the resulting solution was used for the pH adjustment of the preparation to about 6 (Comparative Example 2). When these two ingredients were used in half (Example 3) or one-fourth (Example 2) the amounts used in Comparative Example 2, the dissolution rate was further increased. As shown in Fig. 3, no residual drug or jelly base was observed and favorable drug dissolution was visually confirmed.

### <Example 4 and Comparative Example 3>

Packaged products of a jelly-like preparation were produced in the same manner as in Example 1 using sodium citrate as a basic buffering agent, using citric acid as an acid, and using the amounts of the ingredients shown in Table 2 below.

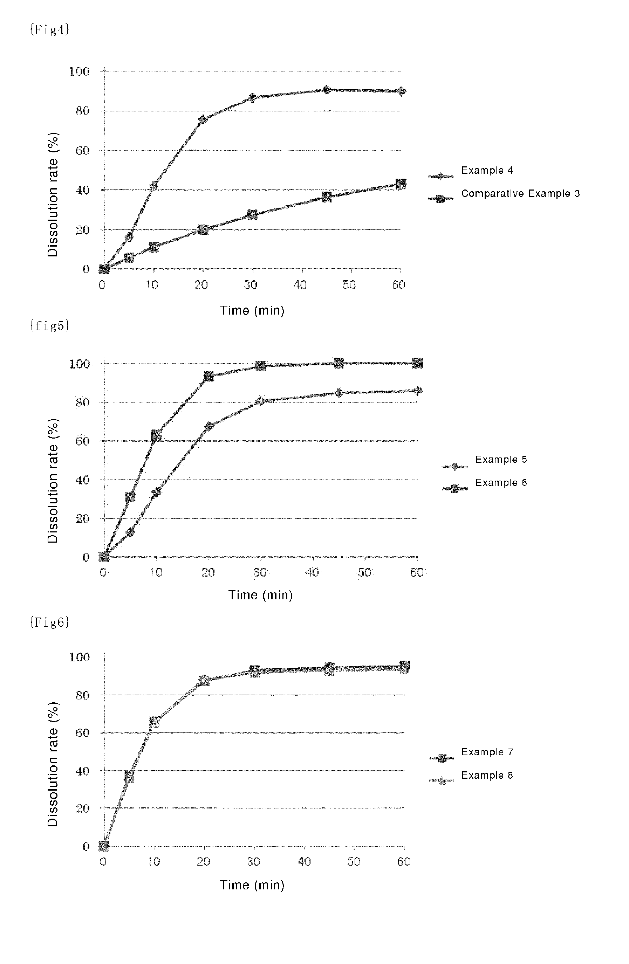

For comparison of the samples of Example 4 and Comparative Example 3, the results of the pH test are shown in Table 2 below, and the results of the dissolution test (in the dissolution medium of pH 1.2) are shown in Fig. 4.

### <Examples 5 and 6>

Packaged products of a jelly-like preparation were produced in the same manner as in Example 1 using disodium hydrogen phosphate as a basic buffering agent, using sodium dihydrogen phosphate as an acid, and using the amounts of the ingredients shown in Table 2 below.

For comparison of the samples of Examples 5 and 6, the results of the pH test are shown in Table 2 below, and the results of the dissolution test (in the dissolution medium of pH 1.2) are shown in Fig. 5.

**Table 2**

| Ingredients | Example 4 | Comparative Example 3 | Example 5 | Example 6 |
|---|---|---|---|---|
| Aripiprazole | 0.30 | 0.30 | 0.30 | 0.30 |
| 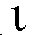 Carrageenan | 0.40 | 0.40 | 0.40 | 0.40 |
| κ Carrageenan | 0.18 | 0.18 | 0.18 | 0.18 |
| Carob bean gum | 0.18 | 0.18 | 0.18 | 0.18 |
| D-sorbitol | 30.0 | 30.0 | 30.0 | 30.0 |
| Glycerin | 10.0 | 10.0 | 10.0 | 10.0 |
| Pluronic F68 | 0.75 | 0.75 | 0.75 | 0.75 |
| Citric acid | 0.020 | 0.080 | - | - |
| Sodium dihydrogen phosphate | - | - | 0.200 | 0.100 |
| Disodium hydrogen phosphate | - | - | 0.180 | 0.090 |
| Sodium citrate | 0.300 | 1.200 | - | - |
| Propyl p-hydroxybenzoate | 0.015 | 0.015 | 0.015 | 0.015 |
| Purified water | 57.855 | 56.895 | 57.795 | 57.985 |
| Total | 100 | 100 | 100 | 100 |
| pH | 6.3 | 6.3 | 6.1 | 6.1 |

(In the table, the amounts of the ingredients are expressed in grams.)

In Fig. 4, citric acid was added to a 0.01 M sodium citrate solution, and the resulting solution was used for the pH adjustment of the preparation to about 6 (Example 4). When these two ingredients were used in 4 times (Comparative Example 3) the amounts used in Example 4, the dissolution rate was reduced. In Fig. 5, sodium dihydrogen phosphate was added to a 0.005 M disodium hydrogen phosphate solution, and the resulting solution was used for the pH adjustment of the preparation to about 6 (Example 5). When these two ingredients were used in half (Example 6) the amounts used in Example 5, the dissolution rate was further increased.

### <Examples 7 and 8>

Packaged products of a jelly-like preparation were produced in the same manner as in Example 1 using sodium citrate as a basic buffering agent, using DL-malic acid as an acid, and using the amounts of the ingredients shown in Table 3 below.

For comparison of the samples of Examples 7 and 8, the results of the pH test are shown in Table 3 below, and the results of the dissolution test (in the dissolution medium of pH 1.2) are shown in Fig. 6.

### <Examples 9 and 10>

Packaged products of a jelly-like preparation were produced in the same manner as in Example 1 using sodium citrate as a basic buffering agent, using sodium dihydrogen phosphate as an acid, and using the amounts of the ingredients shown in Table 3 below.

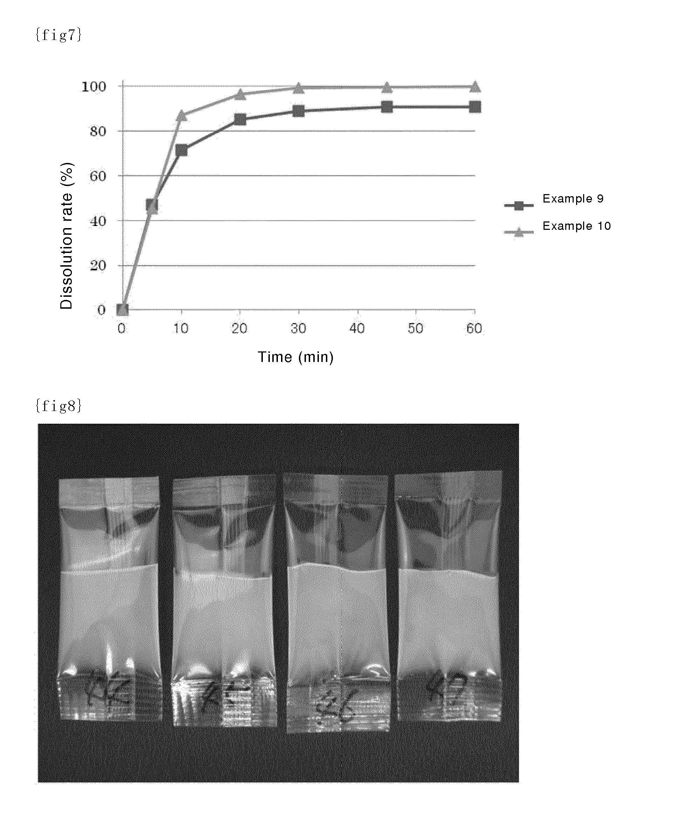

For comparison of the samples of Examples 9 and 10, the results of the pH test and the Brix test (the Brix test was performed on Example 10 only) are shown in Table 3 below, and the results of the dissolution test (in the dissolution medium of pH 1.2) are shown in Fig. 7.

**Table 3**

| Ingredients | Example 7 | Example 8 | Example 9 | Example 10 |
|---|---|---|---|---|
| Aripiprazole | 0.30 | 0.30 | 0.30 | 0.30 |
| 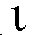 Carrageenan | 0.40 | 0.40 | 0.40 | 0.40 |
| κ Carrageenan | 0.18 | 0.18 | 0.18 | 0.18 |
| Carob bean gum | 0.18 | 0.18 | 0.18 | 0.18 |
| D-sorbitol | 30.0 | 30.0 | 30.0 | 30.0 |
| Glycerin | 10.0 | 10.0 | 10.0 | 10.0 |
| Pluronic F68 | 0.75 | 0.75 | 0.75 | 0.75 |
| DL-malic acid | 0.010 | 0.005 | - | - |
| Sodium dihydrogen phosphate | - | - | 0.100 | 0.050 |
| Sodium citrate | 0.150 | 0.075 | 0.150 | 0.075 |
| Propyl p-hydroxybenzoate | 0.015 | 0.015 | 0.015 | 0.015 |
| Purified water | 58.015 | 58.095 | 57.925 | 58.050 |
| Total | 100 | 100 | 100 | 100 |
| pH | 6.4 | 6.3 | 6.2 | 6.2 |
| Brix% | - | - | - | 39.3 |

(In the table, the amounts of the ingredients are expressed in grams.)

DL-malic acid was added to a 0.005 M sodium citrate solution, and the resulting solution was used for the pH adjustment of the preparation to about 6 (Example 7). As shown in Fig. 6, even when these two ingredients were used in half (Example 8) the amounts used in Example 7, the dissolution rate was still high. In Fig. 7, sodium dihydrogen phosphate was added to a 0.005 M sodium citrate solution, and the resulting solution was used for the pH adjustment of the preparation to about 6 (Example 9). When these two ingredients were used in half (Example 10) the amounts used in Example 9, the dissolution rate was further increased.

### <Example 11>

Packaged products of a jelly-like preparation were produced in the same manner as in Example 1 using disodium hydrogen phosphate as a basic buffering agent, using DL-malic acid as an acid, and using the amounts of the ingredients shown in Table 4 below.

### <Examples 12 to 15>

Packaged products of a jelly-like preparation were produced in the same manner as in Example 11 except for using xylitol, reduced maltose syrup, trehalose or purified sucrose as a sugar instead of D-sorbitol. The photograph of the appearances of the samples of Examples 12 to 15 is shown in Fig. 8 (in the figure, Examples 12, 13, 14 and 15 are shown in the order from the left).

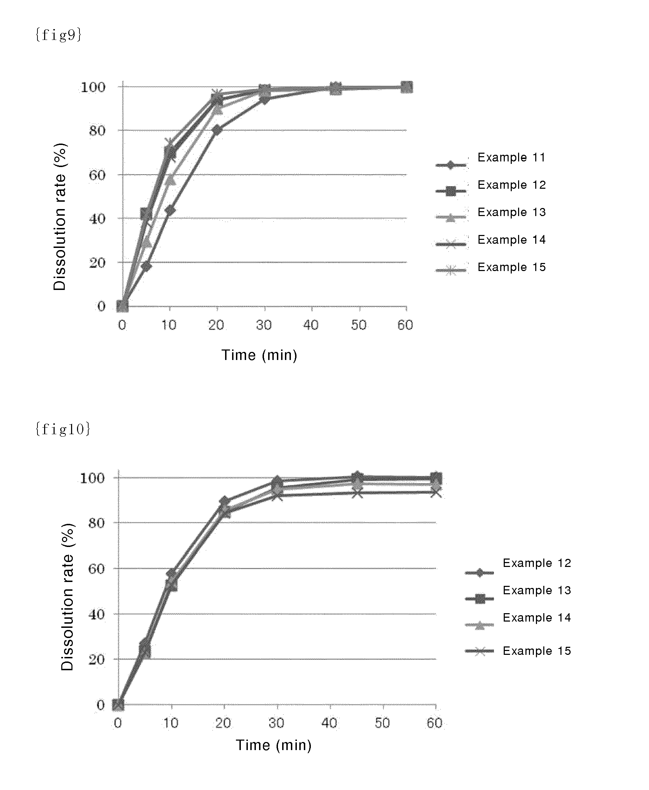

For comparison of the samples of Examples 11 to 15, the results of the pH test and the Brix test are shown in Table 4 below, the results of the dissolution test (in the dissolution medium of pH 1.2) are shown in Fig. 9, and the results of the dissolution test (in the dissolution medium of pH 4.0) are shown in Fig. 10.

**Table 4**

| Ingredients | Example 11 | Example 12 | Example 13 | Example 14 | Example 15 |
|---|---|---|---|---|---|
| Aripiprazole | 0.30 | 0.30 | 0.30 | 0.30 | 0.30 |
| 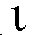 Carrageenan | 0.40 | 0.40 | 0.40 | 0.40 | 0.40 |
| κ Carrageenan | 0.18 | 0.18 | 0.18 | 0.18 | 0.18 |
| Carob bean gum | 0.18 | 0.18 | 0.18 | 0.18 | 0.18 |
| D-sorbitol | 30.0 | - | - | - | - |
| Xylitol | - | 30.0 | - | - | - |
| Reduced maltose syrup | - | - | 30.0 | - | - |
| Trehalose | - | - | - | 30.0 | - |
| Purified sucrose | - | - | - | - | 30.0 |
| Glycerin | 10.0 | 10.0 | 10.0 | 10.0 | 10.0 |
| Pluronic F68 | 0.75 | 0.75 | 0.75 | 0.75 | 0.75 |
| DL-malic acid | 0.025 | 0.025 | 0.025 | 0.025 | 0.025 |
| Disodium hydrogen phosphate | 0.180 | 0.180 | 0.180 | 0.180 | 0.018 |
| Propyl p-hydroxybenzoate | 0.015 | 0.015 | 0.015 | 0.015 | 0.015 |
| Purified water | 57.970 | 57.970 | 57.970 | 57.970 | 57.970 |
| Total | 100 | 100 | 100 | 100 | 100 |
| pH | 6.1 | 6.1 | 5.9 | 6.2 | 6.0 |
| Brix% | 39.3 | 38.0 | 41.0 | 37.7 | 38.1 |

(In the table, the amounts of the ingredients are expressed in grams.)

As shown in Fig. 9, the dissolution rate in the dissolution medium of pH 1.2 slightly varied depending on the kind of sugar, but at 30 minutes after the start of the dissolution test, almost complete dissolution was observed in all the samples. As shown in Fig. 10, there was no difference in the dissolution rate in the dissolution medium of pH 4.0 regardless of the kind of sugar, and almost complete dissolution was observed in all the samples within 30 minute after the start of the dissolution test (Example 11 was tested only in the dissolution medium of pH 1.2).

### <Examples 16 to 19>

Packaged products of a jelly-like preparation were produced in the same manner as in Example 1 using D-sorbitol as a sugar in the amount shown in Table 5 below, using disodium hydrogen phosphate as a basic buffering agent, using DL-malic acid as an acid, and using the amounts of the ingredients shown in Table 5 below.

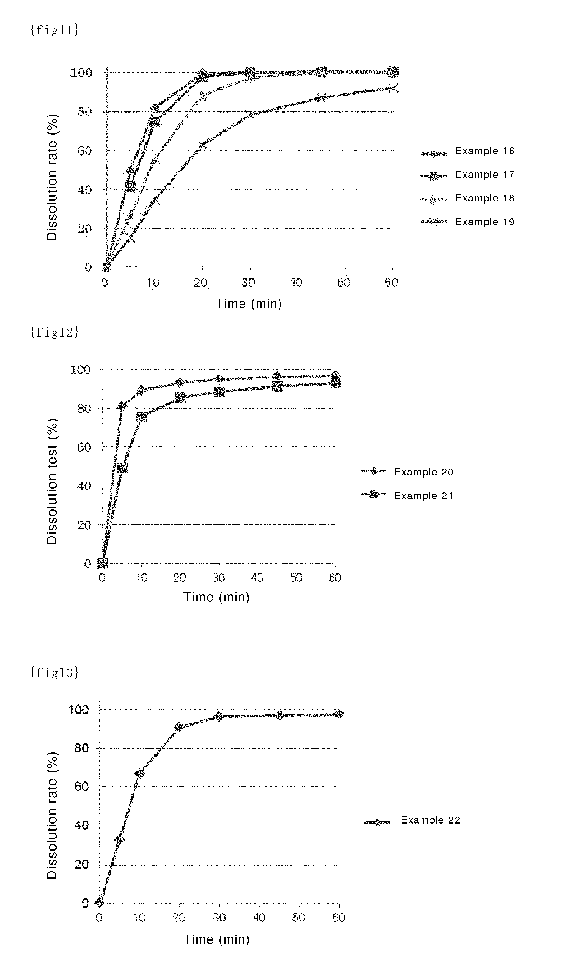

For comparison of the samples of Examples 16 to 19, the results of the pH test and the Brix test are shown in Table 5 below, and the results of the dissolution test (in the dissolution medium of pH 1.2) are shown in Fig. 11.

**Table 5**

| Ingredients | Example 16 | Example 17 | Example 18 | Example 19 |
|---|---|---|---|---|
| Aripiprazole | 0.30 | 0.30 | 0.30 | 0.30 |
| 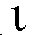 Carrageenan | 0.40 | 0.40 | 0.40 | 0.40 |
| κ Carrageenan | 0.18 | 0.18 | 0.18 | 0.18 |
| Carob bean gum | 0.18 | 0.18 | 0.18 | 0.18 |
| D-sorbitol | 25.0 | 30.0 | 35.0 | 40.0 |
| Glycerin | 10.0 | 10.0 | 10.0 | 10.0 |
| Pluronic F68 | 0.050 | 0.050 | 0.050 | 0.050 |
| DL-malic acid | 0.020 | 0.020 | 0.020 | 0.020 |
| Disodium hydrogen phosphate | 0.150 | 0.150 | 0.150 | 0.150 |
| Propyl p-hydroxybenzoate | 0.015 | 0.015 | 0.015 | 0.015 |
| Purified water | 63.705 | 58.705 | 53.705 | 48.705 |
| Total | 100 | 100 | 100 | 100 |
| pH | 6.2 | 6.2 | 6.3 | 6.2 |
| Brix% | 34.2 | 39.3 | 44.3 | 49.3 |

(In the table, the amounts of the ingredients are expressed in grams.)

In these Examples, DL-malic acid was added to a 0.004 M disodium hydrogen phosphate solution, and the resulting solution was used for the pH adjustment of the preparations to about 6, and the sugar (D-sorbitol) contents of the preparations were adjusted to 25%, 30%, 35% and 40%. The results of the measurement of the dissolution rates are shown in Fig. 11, indicating that the dissolution rate increased as the concentration of the sugar decreased.

### <Examples 20 and 21>

A flowable gelled preparation was produced in the same manner as in Example 1 excluding κ carrageenan from the gelling agents, using disodium hydrogen phosphate as a basic buffering agent, using DL-malic acid as an acid, and using the amounts of the ingredients shown in Table 6 below.

For comparison of the samples of Examples 20 and 21, the results of the pH test and the Brix test are shown in Table 6 below, and the results of the dissolution test (in the dissolution medium of pH 1.2) are shown in Fig. 12.

**Table 6**

| Ingredients | Example 20 | Example 21 |
|---|---|---|
| Aripiprazole | 0.30 | 0.30 |
| 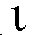 Carrageenan | 0.40 | 0.40 |
| Carob bean gum | 0.18 | 0.18 |
| D-sorbitol | 30.0 | 30.0 |
| Glycerin | 10.0 | 10.0 |
| Pluronic F68 | 0.050 | 0.050 |
| DL-malic acid | 0.050 | 0.100 |
| Disodium hydrogen phosphate | 0.360 | 0.720 |
| Propyl p-hydroxybenzoate | 0.015 | 0.015 |
| Purified water | 58.695 | 57.785 |
| Total | 100 | 100 |
| pH | 6.2 | 6.2 |
| Brix% | 38.7 | 38.4 |

(In the table, the amounts of the ingredients are expressed in grams.)

As shown in Fig. 12, the reduction in the amount of the basic buffering agent resulted in a higher dissolution rate.

### <Example 22>

Packaged products of a jelly-like preparation were produced in the same manner as in Example 1 using sodium citrate as a basic buffering agent, using disodium edetate as an acid, using sucralose as an additional ingredient, and using the amounts of the ingredients shown in Table 7 below.

For comparison of the sample of Example 22, the results of the pH test and the Brix test are shown in Table 7 below, and the results of the dissolution test (in the dissolution medium of pH 1.2) are shown in Fig. 13.

**Table 7**

| Ingredients | Example 22 |
|---|---|
| Aripiprazole | 0.30 |
| 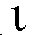 Carrageenan | 0.40 |
| κ Carrageenan | 0.18 |
| Carob bean gum | 0.18 |
| D-sorbitol | 25.0 |
| Glycerin | 10.0 |
| Sucralose | 0.100 |
| Pluronic F68 | 0.050 |
| Disodium edetate | 0.040 |
| Sodium citrate | 0.035 |
| Propyl p-hydroxybenzoate | 0.015 |
| Purified water | 63.700 |
| Total | 100 |
| pH | 6.2 |
| Brix% | 34.1 |

(In the table, the amounts of the ingredients are expressed in grams.)

As shown in Fig. 13, the reduction in the amount of the basic buffering agent resulted in a higher dissolution rate.

### <Examples 23 and 24>

Packaged products of a jelly-like preparation were produced in the same manner as in Example 1 using disodium hydrogen phosphate as a basic buffering agent, using DL-malic acid as an acid, and using the amounts of the ingredients shown in Table 8 below.

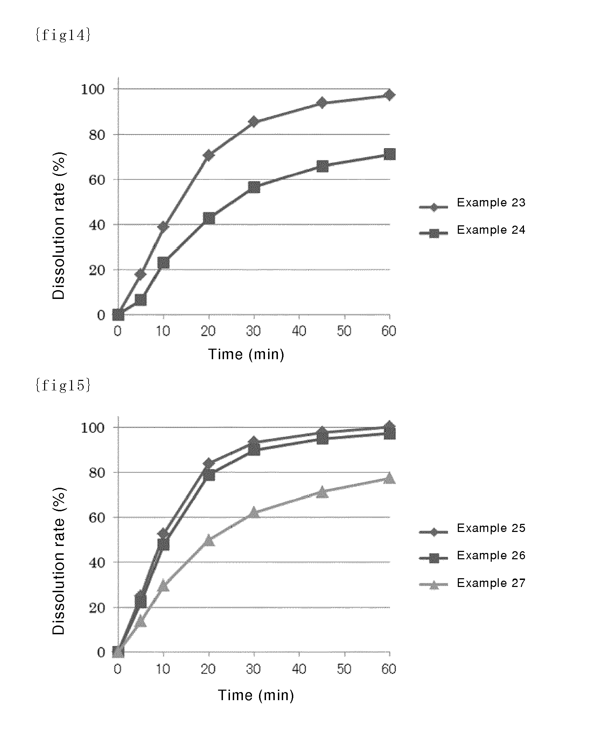

For comparison of the samples of Examples 23 and 24, the results of the pH test and the Brix test are shown in Table 8 below, and the results of the dissolution test (in the dissolution medium of pH 1.2) are shown in Fig. 14.

**Table 8**

| Ingredients | Example 23 | Example 24 |
|---|---|---|
| Aripiprazole | 0.30 | 0.30 |
| 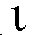 Carrageenan | 0.40 | 0.40 |
| κ Carrageenan | 0.18 | 0.18 |
| Carob bean gum | 0.18 | 0.18 |
| D-sorbitol | 30.0 | 30.0 |
| Glycerin | 10.0 | 10.0 |
| Pluronic F68 | 0.050 | 0.050 |
| DL-malic acid | 0.110 | 0.220 |
| Disodium hydrogen phosphate | 0.360 | 0.720 |
| Propyl p-hydroxybenzoate | 0.015 | 0.015 |
| Purified water | 58.405 | 42.065 |
| Total | 100 | 100 |
| pH | 4.9 | 4.8 |
| Brix% | 38.5 | 38.5 |

(In the table, the amounts of the ingredients are expressed in grams.)

Fig. 14 shows the results of the example in which a reduced amount of DL-malic acid was added to a 0.02 M disodium hydrogen phosphate solution, and the resulting solution was used for the pH adjustment of the preparation to 5 or less (Example 24). As shown in Fig. 14, the reduction of the concentration of the disodium hydrogen phosphate solution to 0.01 M resulted in remarkable improvement in the dissolution rate (Example 23). These results show that, in the case where the pH of the preparation is set to 4.6 or more, the dissolution rate is controlled depending on the amount of the basic buffering agent.

### <Examples 25 to 27>

Packaged products of a jelly-like preparation were produced in the same manner as in Example 1 using disodium hydrogen phosphate as a basic buffering agent, using hydrochloric acid as an acid, and using the amounts of the ingredients shown in Table 9 below.

For comparison of the samples of Examples 25 to 27, the results of the pH test and the Brix test are shown in Table 9 below, and the results of the dissolution test (in the dissolution medium of pH 1.2) are shown in Fig. 15.

**Table 9**

| Ingredients | Example 25 | Example 26 | Example 27 |
|---|---|---|---|
| Aripiprazole | 0.30 | 0.30 | 0.30 |
| 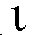 Carrageenan | 0.40 | 0.40 | 0.40 |
| κ Carrageenan | 0.18 | 0.18 | 0.18 |
| Carob bean gum | 0.18 | 0.18 | 0.18 |
| D-sorbitol | 30.0 | 30.0 | 30.0 |
| Glycerin | 10.0 | 10.0 | 10.0 |
| Pluronic F68 | 0.050 | 0.050 | 0.050 |
| HCl (0.1 mol/L) (unit: mL) | 4.5 | 9 | 18 |
| Disodium hydrogen phosphate | 0.180 | 0.360 | 0.720 |
| Propyl p-hydroxybenzoate | 0.015 | 0.015 | 0.015 |
| Purified water | 54.195 | 49.515 | 40.155 |
| Total | 100 | 100 | 100 |
| pH | 6.1 | 5.9 | 5.8 |
| Brix% | 38.2 | 38.3 | 38.5 |

(In the table, the amounts of the ingredients except HCl are expressed in grams. The HCl used was 0.1 moL/L HCl in the amount (unit: mL) shown in the table.)

Hydrochloric acid was added to a 0.02 M disodium hydrogen phosphate solution, and the resulting solution was used for the pH adjustment of the preparation to about 6 (Example 27). As shown in Fig. 15, when these two ingredients were used in half (Example 26) or one-fourth (Example 25) the amounts used in Example 27, the dissolution rate was further increased.

The above oral pharmaceutical preparations of the present invention did not taste sour or bitter even though no masking agent was used therein.

### <Examples 28 to 32>

The ingredients shown in Table 10 below were precisely weighed out. A powder mixture of the gelling agents shown in Table 10 was added to a sugar (D-sorbitol). To this, a basic buffering agent (disodium hydrogen phosphate) and an acid (DL-malic acid) were added, and a preservative (propyl p-hydroxybenzoate) was further added. Further, purified water, concentrated glycerin and aripiprazole were added, and the mixture was heated with stirring in a hot water bath to 80 to 85°C for complete dissolution. After the temperature reached 80°C, with this temperature maintained for 1 hour, the resulting jelly base was sterilized while stirred so gently as not to form bubbles etc. The jelly base was cooled with stirring in a water bath at 20°C (different from the hot water bath mentioned above) to give a thick liquid preparation. If needed for particular experiments, the thick liquid preparation was distributed into previously prepared packaging containers (aluminum stick packs or clear PET containers) so that each container contained a single dosage unit (containing 12 mg of aripiprazole in 4 mL of the preparation), and the containers were then sealed with a sealer to give packaged products of the thick liquid preparation.

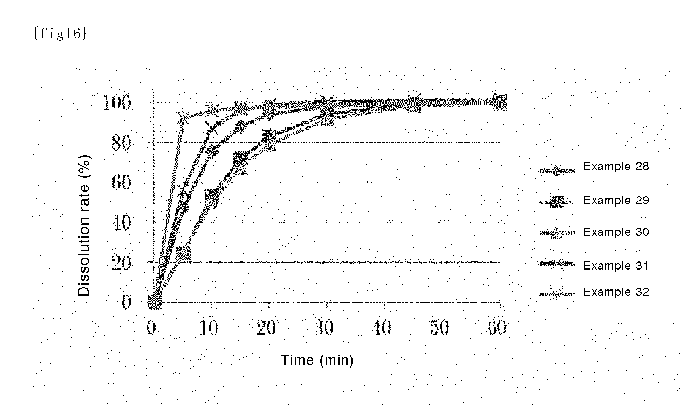

For comparison of the samples of Examples 28 to 32, the results of the pH test, the viscosity measurement test and the appearance observation are shown in Table 10 below, and the results of the dissolution test (in the dissolution medium of pH 4.0) are shown in Fig. 16.

**Table 10**

| Ingredients | Example 28 | Example 29 | Example 30 | Example 31 | Example 32 |
|---|---|---|---|---|---|
| Aripiprazole | 0.30 | 0.30 | 0.30 | 0.30 | 0.30 |
| 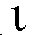 Carrageenan | 0.40 | 0.40 | 0.40 | 0.40 | 0.40 |
| Carob bean gum | 0.10 | 0.10 | 0.10 | 0.10 | 0.10 |
| Agar (low gel strength agar) | 0.10 | - | - | - | - |
| Xanthan gum | - | 0.10 | - | - | - |
| Agar, JP | - | - | 0.10 | 0.050 | - |
| Crystalline cellulose and carmellose sodium | - | - | - | - | 1.00 |
| D-sorbitol | 30.0 | 30.0 | 30.0 | 30.0 | 30.0 |
| Sucralose | 0.020 | 0.020 | 0.020 | 0.020 | 0.020 |
| Glycerin | 10.0 | 10.0 | 10.0 | 10.0 | 10.0 |
| Pluronic F68 | 0.05 | 0.05 | 0.05 | 0.05 | 0.05 |
| DL-malic acid | 0.005 | 0.005 | 0.005 | 0.005 | 0.005 |
| Disodium hydrogen phosphate | 0.038 | 0.038 | 0.038 | 0.038 | 0.038 |
| Propyl p-hydroxybenzoate | 0.015 | 0.015 | 0.015 | 0.015 | 0.015 |
| Purified water | 58.972 | 58.972 | 58.972 | 59.022 | 58.072 |
| Total | 100 | 100 | 100 | 100 | 100 |
| pH | 6.4 | 6.4 | 6.5 | 6.4 | 6.3 |
| Viscosity at 20°C (mPa·s) (shear rate: 50 sec⁻¹) | 73.3 | 150.1 | 125.5 | 110.8 | 29.9 |
| Appearance (dispersion stability) | No sediments | No sediments | No sediments | No sediments | No sediments |

(In the table, the crystalline cellulose and carmellose Na is CEOLUS RC-A591NF (manufactured by Asahi Kasei Chemicals Corporation), which is a mixture of 89% of crystalline cellulose and 11% of carmellose Na.)

As described above, the oral pharmaceutical preparation of the present invention does not taste sour or bitter even though no masking agent is used therein. In addition, the oral pharmaceutical preparation is excellent in dissolution rate as shown in the figures, and thus the content ratio of the medicinal ingredient (aripiprazole) in the preparation can be increased and the volume of the preparation as a whole can be reduced. Therefore, medication burden on patients can be reduced. Further, the oral pharmaceutical preparation of the present invention, due to its dosage form, enables patients to easily confirm that they have successfully swallowed the preparation.

### INDUSTRIAL APPLICABILITY

The aripiprazole-containing oral pharmaceutical preparation of the present invention is a useful antipsychotic that has suppressed sourness and bitterness, imposes less medication burden on patients, has a higher dissolution rate, and enables patients to easily confirm that they have successfully swallowed the preparation.

## Claims

1. An oral pharmaceutical preparation comprising aripiprazole and a gelling agent and having a pH of 4.6 or more.

2. The oral pharmaceutical preparation according to claim 1, wherein the pH of the preparation is adjusted by use of a pharmaceutically usable basic buffering agent at a molar concentration of 0.03 M or less.

3. The oral pharmaceutical preparation according to claim 2, wherein the basic buffering agent is one or more kinds selected from the group consisting of citrates or their hydrates, malates or their hydrates, phosphates or their hydrates and edetates or their hydrates.

4. The oral pharmaceutical preparation according to claim 2, wherein the basic buffering agent is one or more kinds selected from the group consisting of sodium citrate, disodium citrate, trisodium citrate, sodium DL-malate, trisodium phosphate, disodium hydrogen phosphate, dipotassium hydrogen phosphate and tetrasodium edetate.

5. The oral pharmaceutical preparation according to any one of claims 1 to 4, wherein the pH of the preparation is adjusted by use of a pharmaceutically usable basic buffering agent at a molar concentration of 0.03 M or less and a pharmaceutically usable acid.

6. The oral pharmaceutical preparation according to any one of claims 1 to 5, further comprising a sugar at a concentration of 50 W/W% or less.

7. The oral pharmaceutical preparation according to any one of claims 1 to 6, wherein the preparation is a semisolid preparation.

8. The oral pharmaceutical preparation according to any one of claims 1 to 6, wherein the preparation is a liquid preparation.

9. The oral pharmaceutical preparation according to claim 8, wherein the liquid preparation is a thick liquid preparation.

10. The oral pharmaceutical preparation according to any one of claims 1 to 9, wherein the gelling agent is one or more kinds selected from the group consisting of gum arabic, gum arabic powder, alginic acid, sodium alginate, propylene glycol alginate, carrageenan, karaya gum powder, carmellose sodium, crystalline cellulose and carmellose sodium, carob bean gum, curdlan, agar, agar powder, xanthan gum, guar gum, psyllium seed gum, gellan gum, purified gelatin, gelatin, tamarind seed gum, tara gum, tragacanth, tragacanth powder, furcellaran, pullulan and pectin.

11. A method for producing the oral pharmaceutical preparation according to any one of claims 1 to 10, comprising adding a gelling agent to aripiprazole and further adding a pharmaceutically usable basic buffering agent so that the pH of the preparation is adjusted to 4.6 or more.

12. An oral pharmaceutical composition for use in treatment of mental disorders, the composition comprising aripiprazole and a gelling agent and having a pH of 4.6 or more.

13. A method for treating mental disorders using an oral pharmaceutical composition comprising aripiprazole and a gelling agent and having a pH of 4.6 or more.
